# EUROPÄISCHE PATENTSCHRIFT

(11) **EP 1 414 523 B1**
(45) Veröffentlichungstag und Bekanntmachung des Hinweises auf die Patenterteilung: **15.04.2009**
(21) Anmeldenummer: 02747470.9
(22) Anmeldetag: 22.07.2002
(51) Int. Cl.: A61K 31/191, A61K 33/06, A61P 9/10

(54) **VERWENDUNG EINES LACTATSALZES ZUR BEHANDLUNG UND PROPHYLAXE DER ATHEROSKLEROSE**
USE OF A LACTATE SALT FOR THE TREATMENT AND PROPHYLAXIS OF ATHEROSCLEROSIS
UTILISATION D'UN SEL DE LACTATE POUR LE TRAITEMENT ET LA PROPHYLAXIE DE L'ATHEROSCLEROSE

(30) Priorität: 20.07.2001 DE 10135494
(43) Veröffentlichungstag der Anmeldung: 06.05.2004
(73) Patentinhaber: "S.u.K." Beteiligungs GmbH, 1010 Wien (AT)
(72) Erfinder: KRAUSKOPF, Jobst, 29576 Barum (DE); ELSTNER, Erich, 82194 Gröbenzell (DE)
(74) Vertreter: HOFFMANN EITLE
(86) Internationale Anmeldenummer: PCT/EP2002/008153
(87) Internationale Veröffentlichungsnummer: WO 2003/011263

(56) Entgegenhaltungen:
- EP-A- 0 274 404
- WO-A-00/67750
- WO-A-99/65337
- US-A- 4 412 986
- US-A- 6 042 849
- DATABASE WPI Section Ch, Week 200132 Derwent Publications Ltd., London, GB; Class B04, AN 2001-300860 XP002237124 & CN 1 282 536 A (LIU G), 7. Februar 2001 (2001-02-07)
- DATABASE WPI Section Ch, Week 199729 Derwent Publications Ltd., London, GB; Class B05, AN 1997-311237 XP002247268 & CN 1 104 625 A (ZHANG J), 5. Juli 1995 (1995-07-05)
- DATABASE WPI Section Ch, Week 200110 Derwent Publications Ltd., London, GB; Class B04, AN 2001-081199 XP002247269 & CN 1 145 185 A (CHEN D), 19. März 1997 (1997-03-19)
- PATENT ABSTRACTS OF JAPAN vol. 016, no. 431 (C-0983), 9. September 1992 (1992-09-09) & JP 04 148651 A (NORIHIRO KAMATA), 21. Mai 1992 (1992-05-21)
- DATABASE CA [Online] CHEMICAL ABSTRACTS SERVICE, COLUMBUS, OHIO, US; HAGIWARA, SATOSHI: "Treatment for osteoporosis" retrieved from STN Database accession no. 127:12760 HCA XP002247267 & SHOJINKAI IGAKUSHI ( 1996 ), 35(2), 65-70 ,
- "Milk tea with VA, VD and calcium lactate" DERWENT, XP002237125

## Beschreibung

### Gebiet der Erfindung

Die vorliegende Erfindung betrifft die Verwendung von Calcium-Lactat zur Behandlung und Prophylaxe der Atherosklerose.

### Hintergrund der Erfindung

Eine der bedeutsamsten und häufigsten Erkrankungen in der westlichen Welt ist die Atherosklerose, umgangssprachlich auch als Arterienverkalkung bekannt. Ihre Ätiopathogenese konnte noch nicht vollständig geklärt werden. So werden zahlreiche exogene und endogene Noxen bzw. Krankheiten für die Auslösung bzw. Förderung der Atherosklerose verantwortlich gemacht. Beispiele davon sind Hypertonie, Hyperlipidämie, Hyperfibrinogenämie, Diabetes mellitus, Toxine, Nicotin, Antigen-Antikörper-Komplexe, Entzündungen, usw. Ein erhöhter Lipidblutspiegel, insbesondere die Hypercholesterinämie, d.h. ein erhöhter Cholesterinblutspiegel (> 200 mg/dl), ist fraglos ein bedeutsamer Risikofaktor. Ein sinnvoller Ansatzpunkt für die Prophylaxe sowie in gewissem Umfang auch für die Therapie dieser Erkrankung und ihrer Folgen (Herzinfarkt, zerebrale und periphere Durchblutungsstörungen, u.a.) besteht bisher darin, erhöhte Plasmalipidspiegel, insbesondere erhöhte Plasmacholesterinspiegel zu senken.

In gleicher Weise wie andere Arterien können auch die Koronararterie, und zwar vor allem die größeren Koronararterienäste, von einer Atherosklerose betroffen sein. Die Koronarsklerose, die zu einer Einengung der koronaren Strombahn oder durch zusätzlich Thrombenbildung zu teilweisem oder vollständigem Verschluß von Koronararterienästen führt ist die wichtigste Ursache der koronaren Herzkrankheit. Als Risikofaktoren sind gesichert: Rauchen, Übergewicht, Hypertonie, Hyperlipoproteinämie und Diabetes mellitus. Bei der Prophylaxe sind diese Faktoren zu berücksichtigen.

Als Plasmalipide kommen Neutralfette, Phospholipide, Cholesterin, Cholesterinester und freie Fettsäuren vor. Da Lipide wasserunlöslich sind, werden sie im Blut nicht in freier Form, sondern in Form sogenannter Lipoproteine, d.h. gebunden an Trägerproteine, transportiert. Die Lipoproteine werden unterteilt in Chylomikrone, Very-low-density-Lipoproteine (VLDL), Low-density-Lipoproteine (LDL), Intermediate-density-Lipoproteine (IDL) und High-density-Lipoproteine (HDL). Während die LDL als Hauptbestandteil Cholesterin aufweisen, besitzen die HDL einen besonders hohen Protein- und einen verhältnismäßig niederen Cholesteringehalt. Die HDL sind in der Lage, in Gefäßwände abgelagertes Cholesterin aufzunehmen und es an die IDL abzugeben.

Chylomikronen entstehen bei der Fettresorption in der Darmwand, gelangen dann auf dem Lymphweg ins Blut und geben nach Spaltung der Triglyceride durch die Lipoproteinlipase, den sog. Klärfaktor, Fettsäuren an das Fettgewebe (zur Speicherung) und an die Muskulatur (als Brennstoff) ab. Die verbleibenden Überreste (Remnants), die einen hohen Gehalt an Cholesterinestern aufweisen, werden nun an einen speziellen Rezeptor (Remnant-Rezeptor), der nur in Leberzellen vorkommt, gebunden und durch diesen in die Leberzelle eingeschleust. Das in die Leberzelle aufgenommene Cholesterin wird einerseits in Gallensäuren umgewandelt, die dann mit der Galle in den Darm abgegeben werden, andererseits zusammen mit Apoproteinen, Phospholipiden und Triglyceriden in Form von VLDL wieder in den Kreislauf eingeschleust.

CN 1104625 offenbart die Verwendung von Chromium-, Kupfer- und Mangan-Laktat zur Vorbeugung und Behandlung von kardiovaskulären Erkrankungen.

Die Aufgabe der vorliegenden Erfindung liegt nun darin, eine pharmazeutische Zusammensetzung bereitzustellen, die zur Behandlung und/oder Prophylaxe der Atherosklerose und/oder zur Prophylaxe oder Behandlung von durch Atherosklerose verursachten Krankheiten in der Lage ist.

### Beschreibung der Erfindung

Es wurde überraschend gefunden, daß die Verabreichung von Calcium-Lactat zu einer Verminderung des Atherosklerose führt.

Lactat ist das Salz der Milchsäure und existiert in der L-(+)-Form (rechtsdrehend), der D-(-)-Form und der DL-Form (Racemat). Als besonders vorteilhaft hat sich die Verwendung der L-Form erwiesen.

Nach intensiven und detaillierten Studien haben die Erfinder die Entdeckung gemacht, dass in der unmittelbaren Nachbarschaft von atherosklerotischen Ereignissen in den Blutgefässen Lactat-Dehydrogenase von beschädigten Endothelzellen freigesetzt wird, wodurch Lactat in Pyruvat umgewandelt werden kann. Pyruvat als eine α-Ketosäure ist in der Lage mit bestimmten Peroxiden zu reagieren, wodurch diese toxischen Verbindungen von der reaktiven Plattform entfernt und somit dem atherosklerotischen Mechanismus entzogen werden. Durch Gabe von Calcium-Lactat kann nun dieser Mechanismus verstärkt unterdrückt und das Atheroskleroserisiko verringert, bestenfalls vollständig unterdrückt werden.

Das gleich trifft auf die inflammatorischen Stellen zu: die Bildung von Hypochlorit aus Chlorid und Wasserstoffperoxid durch die Myeloperoxidase-Reaktion aktivierter neutrophiler Granulozyten ist ein gewebeschädigender und -reizender Pfad. Die ionische Reaktion von Peroxid mit Pyruvat sollte ebenso dieses irritierende und toxische Potential dieser aktivierten Blutzellen ausschalten.

Calcium ist dabei in der Lage, die über den enterohepatischen Kreislauf in den Darm gelangten Gallensäuren zu binden. Dadurch wird der Rücktransport in die Leber vermindert. Zum Aufrechterhalten des Gleichgewichts im enterohepatischen Kreislauf muß dadurch in der Leber vermehrt Cholesterin in Gallensäure umgewandelt werden. Es kommt zu einer Absenkung des Cholesteringehalts im Plasma. Somit wird neben der Einwirkung auf das Entstehen von sklerotischen Arterien gleichzeitig ein möglicher Hauptverursacher für die Atherosklerose dem Körper entzogen, was das AtheroskleroseRisiko noch weiter vermindert.

Calcium ist ein wichtiger Nährstoff für normales Wachstum und Entwicklung. Es hilft bei der Regulierung der Zellfunktion und ist ein unverzichtbarer Strukturbaustein des Knochens. Da der Körper Calcium nicht selbst herstellen kann, muss er es über die Ernährung aufnehmen. Etwa 25 bis 35 % des durch die Nahrung aufgenommenen Calciums werden im Darm, überwiegend im Zwölffingerdarm und Leerdarm, absorbiert, die restlichen 65 bis 75 % des zugeführten Calciums werden nicht genutzt und somit ausgeschieden. Somit stehen etwa 2/3 bis 3/4 des aufgenommenen Calciums zum Binden der Gallensäuren und somit zur Senkung des Cholesterinspiegels zur Verfügung. Die absorbierte Menge führt hingegen zur einer Verbesserung des Knochengerüsts und somit Vorbeugung von Osteoporose.

Calcium-Lactat zeichnet sich durch eine gute Löslichkeit aus. Dadurch kann das Calcium besser absorbiert werden, was die positive Auswirkung auf das Knochengerüst erhöht. Dadurch wird zwar der Anteil der im Darm absolut zur Verfügung stehenden Calciummenge verringert, dem kann jedoch einfach durch Erhöhung der aufgenommenen Menge entgegengewirkt werden. Außerdem ist die Calciummenge im menschlichen Körper ein kontinuierlicher Ausgleich zwischen Absorption und Ausscheidung. Das vermehrt aufgenommene Calcium wird somit zum größten Teil wieder ausgeschieden und kann möglicherweise die Gallensäurenbindung und somit -ausscheidung durch Wirkung von innen noch verstärken.

In einer weiteren erfindungsgemäßen Ausführungsform hat sich eine Mikroverkapselung des Calcium-Lactats bzw. einer daraus hergestellten pharmazeutischen Zusammensetzung als besonders vorteilhaft erwiesen. Die Mikroverkapselung kann z.B. wie in den Patent-Offenlegungsschriften DE 198 54 749 A1 und DE 100 08 880 A1 und dem Gebrauchsmuster DE 296 23 285 U1 beschrieben, erfolgen. Dabei wird das Lactatsalz zum Beispiel in einer Hülle aus einem Polysaccharid, wie z.B. Alginat, fest eingeschlossen. Damit der möglicherweise unverdauliche Hüllstoff eine Freisetzung des Lactats nicht verhindert und dadurch eine ernährungsphysiologische Nutzung durch den Organismus unmöglich macht kann eine verdauliche Komponente, wie z.B. Stärke der Umhüllung beigefügt werden. Durch geschickte Wahl und/oder Kombination der löslichen und unlöslichen Umhüllungskomponenten kann so die Abgabe des mikroverkapselten Lactatsalzes in verschiedenen Bereichen des Verdauungstrakts gezielt gesteuert werden. Eine abgestufte Freisetzung des Lactats im Darm, z.B. eine Freisetzung von 50 bis 80 Gew.-%, bevorzugt von 60 bis 70 Gew.-%, insbesondere von 62,5 Gew.-% des Lactats im Dünndarm, und eine Freisetzung von 20 bis 50 Gew.-%, bevorzugt von 30 bis 40 Gew.-%, insbesondere von 62,5 Gew.-% des Lactats im Dickdarm ist ein mögliche Art der gezielten Freisetzung. Ein weiterer vorteilhafter Effekt kann durch eine verlängerte Haltbarkeit durch Schutz der verkapselten Präparate z.B. vor Umwelteinflüssen erzielt werden.

In Form des Calcium-Lactats ist die tägliche Dosis üblicherweise etwa 2,5 bis 7,0 g, bevorzugt zwischen 4,0 und 6,0 g, am bevorzugtesten 5,0 g.

Die Zusammensetzung dient bevorzugt zur oralen Gabe. Die Zusammensetzung kann in fester Form als Lutschtablette, Pulver oder Granulat, oder in flüssiger Form als Sirup oder Saft vorliegen.

Die Verabreichung geschieht vorteilhafterweise in Dosierungen von einmal bis sechsmal täglich, wobei eine vier- bis sechsmal tägliche Dosis bevorzugt ist. Das tatsächliche Dosisintervall und die Dosismenge hängt jedoch ab von Faktoren wie z.B. Alter, Gewicht, und/oder Geschlecht, die von Individuum zu Individuum variieren können. Insbesondere bei Schwangeren und stillenden ist eine erhöhte Einnahmemenge des Lactats vorteilhaft. Ein zusätzlicher vorteilhafter Einfluß kann sich z.B. auch durch Einnahme des Präparats kurz vor oder während der Mahlzeiten ergeben. Bei der Verabreichung des Lactatsalzes an Säuger im allgemeinen erfolgt die Dosierung ebenfalls in Abhängigkeit von Tierart und Gewicht.

Erfindungsgemäß kann Calcium-Lactat verwendet werden zur Herstellung von Lebensmitteln, wie z.B. diätetischen Lebensmitteln und/oder Nahrungsergänzungsmitteln, wodurch diese die Eigenschaft erhalten, daß sie bei oraler Verabreichung eine Verringerung des Atherosklerose-Risikos bewirken können. In Form des Calcium-Lactats kann zusätzlich noch eine Absenkung des Cholesterinsgehalts bewirkt werden. Beispiele für derartige Lebensmittel sind mit dem Lactat angereicherte Milchprodukte oder Fruchtsäfte.

Ferner ist das Calcium-Laktat verwendbar zur Prophylaxe und/oder zur Behandlung von Krankheiten wie z.B. Osteoporose, Bluthochdruck, entzündlichen Vorgängen wie Arthritis, usw. bei Säugern, insbesondere beim Menschen. Bevorzugt ist die Prophylaxe bzw. Behandlung von koronaren Herzkrankheiten.

## Patentansprüche

1. Verwendung von Calcium-Lactat zur Herstellung einer pharmazeutischen Zusammensetzung zur Prophylaxe und/oder Behandlung von Atherosklerose und/oder zur Prophylaxe oder Behandlung von durch Atherosklerose verursachten Krankheiten.

2. Verwendung gemäß Anspruch 1, wobei das Calcium-Lactat Calcium-L-Lactat ist.

3. Verwendung gemäß Anspruch 1 oder 2, worin die pharmazeutische Zusammensetzung zur gleichzeitigen Verbesserung des Knochengerüsts und/oder zur gleichzeitigen Prophylaxe und/oder Behandlung von Osteoporose dient.

4. Verwendung gemäß einem der Ansprüche 1 bis 3, worin die Zusammensetzung mikroverkapselt ist.

5. Verwendung gemäß einem der Ansprüche 1 bis 4, worin die Zusammensetzung zur oralen Gabe dient.

6. Verwendung gemäß einem der Ansprüche 1 bis 5, worin die tägliche Dosis zwischen 2,5 g und 7,0 g, bevorzugt zwischen 4,0 g und 6,0 g, am bevorzugtesten 5,0 g Calcium-Lactat beträgt.

7. Verwendung gemäß einem der Ansprüche 1 bis 6, wobei die durch Atherosklerose verursachten Krankheiten koronare Herzkrankheiten sind.

8. Verwendung gemäß einem der Ansprüche 1 bis 7, wobei die Zusammensetzung ferner Zusatzstoffe enthält, die zur Herstellung von Lebensmitteln, insbesondere diätetischen Lebensmitteln, oder Nahrungsergänzungsmitteln geeignet sind.

9. Verwendung gemäß einem der Ansprüche 1 bis 8, wobei die Zusammensetzung ferner pharmazeutisch annehmbare Zusatzstoffe und/oder Trägermaterialien umfasst.

10. Verwendung gemäß einem der Ansprüche 1 bis 9, wobei die Zusammensetzung in fester Form als Lutschtablette, Pulver oder Granulat, oder in flüssiger Form als Sirup oder Saft vorliegt.

## Claims

1. Use of calcium lactate for preparing a pharmaceutical composition for the prophylaxis and/or treatment of atherosclerosis and/or for the prophylaxis or treatment of diseases caused by atherosclerosis.

2. Use according to claim 1, wherein the calcium lactate is calcium L-lactate.

3. Use according to claim 1 or 2, wherein the pharmaceutical composition serves the simultaneous improvement of the skeleton and/or the simultaneous prophylaxis and/or treatment of osteoporosis.

4. Use according to any of claims 1 to 3, wherein the composition is microencapsulated.

5. Use according to any of claims 1 to 4, wherein the composition is for oral administration.

6. Use according to any of claims 1 to 5, wherein the daily dose is between 2.5 g and 7.0 g, preferably between 4.0 g and 6.0 g and most preferably 5.0 g of calcium lactate.

7. Use according to any of claims 1 to 6, wherein the diseases caused by atherosclerosis are coronary heart diseases.

8. Use according to any of claims 1 to 7, wherein the composition further contains additives suitable for preparing foods, in particular dietetic foods, or food supplements.

9. Use according to any of claims 1 to 8, wherein the composition further comprises pharmaceutically acceptable additives and/or carrier materials.

10. Use according to any of claims 1 to 9, wherein the composition is present in solid form as a lozenge, powder or granular material or in liquid form as a syrup or juice.

## Revendications

1. Utilisation de lactate de calcium pour la préparation d'une composition pharmaceutique pour la prophylaxie et/ou le traitement de l'athérosclérose et/ou pour la prophylaxie ou le traitement de maladies engendrées par l'athérosclérose.

2. Utilisation selon la revendication 1, où le lactate de calcium est le L-lactate de calcium.

3. Utilisation selon la revendication 1 ou 2, où la composition pharmaceutique sert à l'amélioration simultanée de la structure osseuse et/ou pour la prophylaxie et/ou le traitement simultané de l'ostéoporose.

4. Utilisation selon l'une des revendications 1 à 3, où la composition est microencapsulée.

5. Utilisation selon l'une des revendications 1 à 4, où la composition sert à une administration orale.

6. Utilisation selon l'une des revendications 1 à 5, où la dose quotidienne se situe dans l'intervalle allant de 2,5 g à 7,0 g, de préférence de 4,0 g à 6,0 g et de manière la plus préférée, à 5,0 g de lactate de calcium.

7. Utilisation selon l'une des revendications 1 à 6, où les maladies engendrées par l'athérosclérose sont des maladies cardiaques coronaires.

8. Utilisation selon l'une des revendications 1 à 7, où la composition contient en outre, des additifs, qui sont appropriés pour la préparation d'aliments, en particulier d'aliments diététiques, ou de compléments alimentaires.

9. Utilisation selon l'une des revendications 1 à 8, où la composition comprend en outre, des additifs et/ou supports pharmaceutiquement acceptables.

10. Utilisation selon l'une des revendications 1 à 9, où la composition se présente sous forme solide, en tant que comprimés à sucer, poudre ou granulé, ou sous forme liquide, en tant que sirop ou jus.
